# EUROPEAN PATENT APPLICATION

(11) **EP 3 214 162 A1**
(43) Date of publication of application: **06.09.2017**
(21) Application number: 17159170.4
(22) Date of filing: 03.03.2017
(51) Int. Cl.: C12G 1/022, C12M 1/00, B65D 90/00, C12G 1/02

(54) **PRESSURE CONTROL METHOD AND APPARATUS FOR A FERMENTATION TANK**

(30) Priority: 03.03.2016 IT UA20161324
(71) Applicant: Noform S.r.l., 30020 Meolo (VE) (IT)
(72) Inventor: CROSATO, Stefano, 30020 MEOLO (VE) (IT)
(74) Representative: Citron, Massimiliano

(57) **Abstract**

To reduce the risk of implosion of a tank (10) capable of fermenting a vegetable product in pressurized environment, a method is provided with the steps of
equipping the tank with a device (36) that allows the entry of outer gas inside the tank if the internal pressure of the tank is lower than that of the outer gas, and
providing two operation modes of the tank:
- a first mode wherein the device (36) and the tank (10) are in communication with each other to allow the potential entry of gas inside the tank through the device; and
- a second mode wherein the device (36) and the tank (10) are mutually isolated.

## Description

The invention relates to a control method and apparatus for the internal pressure of a fermentation tank, and in particular it relates to a fermentation tank so controlled and a device that implements the method. The description that follows will make reference by example to winemaking, a sector in which the invention has proven particularly effective.

The implosion of tanks is a known and unfortunately expensive event for the damages it can create, like also reported in the introduction of US 4736864. For example autoclaves, especially for the sector of wine or beer fermentation, are built with resistant but light structure, to contain costs and weights. Therefore a small internal vacuum (40-60 mbar) is sufficient to destroy them, and more or less of a few negative millibars can make the difference between a disaster and a surviving autoclave. Usually the phenomenon is fought with a vacuum-breaker valve, but the problem is actually solved only partially. As also described in US 5071021 col. 1 lines 36-40, the internal mechanism of the vacuum-breaker valve sometimes jams, and is not very reliable especially if the internal pressure of the tank, as in autoclaves, is very high. It is therefore a fact that a vacuum-breaker valve that (a) is applicable to an autoclave or tank whose internal pressure can easily reach 3 or 8 bar and that (b) opens with certainty and precision for a vacuum of -20 or -30 millibars, does not exist.

The present invention has as main object to improve this state of art.

In particular, the invention has as its object the realization of a method for avoiding, or at least mitigating, the danger of implosion for a tank, in particular for an autoclave, in which the internal pressure reaches at least relative +0.5 bar (the external pressure). The method to reduce the risk of implosion of a tank capable of fermenting a vegetable product in pressurized environment, comprises the steps of
equipping the tank with a device that allows (e.g. spontaneously) the entry of outer gas inside the tank if the internal pressure of the tank is lower than that of the outer gas with a maximum pressure difference to 50 millibars (in particular, but not necessarily, the external gas is the atmosphere and the external pressure is the atmospheric one), and
providing two operation modes of the tank:
- a first mode wherein the device (36) and the tank (10) are in communication with each other to allow the potential entry of gas inside the tank through the device; and
- a second mode wherein the device (36) and the tank (10) are mutually isolated.

Let P be the internal pressure of the tank and Pgas that of the external gas. The device allows the entry of external gas if Pgas > P or at most with a maximum difference of 50 millibars between P and Pgas, namely the device surely intervenes or has already intervened when
Pgas - P = 50 millibar.

Thanks to the method the device is isolated from - and is not affected by - the high operating pressure inside the tank, and does not risk jamming. Also, one can perform maintenance to the device during the second mode.

Here are some advantageous variants of the method, usable alone or in combination with each other:
- a selection command (namely a command to set into communication with or isolate from each other the device and the tank) of the two operating modes may be acquired from a user (e.g. by means of a mechanical button or a touch-screen), in order to control manually the mode the tank operates in. This is useful e.g. to coordinate or to best manage the various processing phases; and/or
- the management of the two modes can be automated, to prevent human error. To this aim, the method provides the step of detecting the internal pressure of the tank during the second mode and automatically switch to the first mode if the detected pressure is below a first threshold (e.g. the 1st threshold may be a value between 0 and -30 millibar); and/or
- the internal pressure of the tank may be detected during the second mode and an alarm signal generated if the detected pressure is below a 2nd threshold (e.g. the 2nd threshold may be a value between 0 and -30 millibar). Preferably the 1st and 2nd threshold are different, to allow a user to intervene in a differentiated manner.

To facilitate interaction with the system's devices, preferably the pressure is detected (i) inside a duct that from the top of the tank extends down to its base or (ii) on the top of the tank. Often the duct is already present or by putting it one avoids installing it on top of the tank, where it is inconvenient or dangerous to operate.

Preferably the method has the step of detecting/ controlling the presence of liquid at the point where the pressure is detected, and in affirmative case evacuating the liquid from the duct to eliminate the contact with the liquid for said point, optionally also with the step of isolating the duct from the tank. The advantage is to avoid both reading an incorrect value of pressure inside the duct, and that, by expelling the liquid, the tank implodes.

As another safety against the implosion, a normally-open valve may be supplied to hold it closed, the valve being placed on the top of the tank and interposed between the tank interior and a diaphragm adapted to break if the internal pressure of the tank is lower than that of the external gas. The diaphragm is suitably calibrated and can break only once, then needing substitution. The normally-open valve is then kept closed and is opened only in the event of power supply failure, i.e. in exceptional conditions and symptom of severe fault, in which case the mechanical pressure control performed by the diaphragm justifies its probable rupture in order to safeguard the tank from implosion.

Preferably, said device comprises or is constituted by a valve, for simplicity of construction. The valve does not need to be sophisticated, thanks to the protection ensured by the two above-described modes.

Preferably, for economy, efficiency or ease to program them, the phases of the methods defined here are managed and/or executed and/or programmable through a software program and a microprocessor by which the software is executed. In particular, the program executes some of the abovementioned variants by the steps of
- acquiring from a sensor an electrical signal concerning the internal pressure of the tank [or the level of the liquid in the vertical duct],
- determining from the electrical signal a pressure [or liquid level] data;
- comparing the data with a pressure [or liquid] threshold;
- depending on the comparison result, switching from a mode to the other [or commanding the emptying of the vertical duct].

Another variant envisages a method to prevent the implosion of a tank capable of fermenting a plant product in pressurized environment, with the steps of:
- acquiring an electrical signal from a sensor relative to the internal pressure of the tank,
- determining from the electrical signal a pressure data,
- comparing the data with a null or negative pressure threshold,
- putting the tank into communication with the external environment if the data is equal to or less than the threshold.

Another aspect of the invention relates to a tank for fermenting a plant product in pressurized environment, comprising
a pressurizable container,
a first device for allowing entry of outside air inside the container if the internal pressure of the container is lower than the atmospheric pressure with a maximum pressure difference of 50 millibars,
a second device operable to selectively put into communication or isolate the interior of the container and the first device.

This tank implements the method and has the advantages thereof. As advantageous variants of the tank for fermenting:
- the first device, for simplicity of construction and to avoid implosion, comprises or is constituted by a valve intended to open if the external pressure is greater than that inside to container, preferably with a maximum pressure difference equal to 50 millibars; and/or
- the first device comprises or is constituted by a valve intended to open if the pressure inside the container is greater than a positive pressure threshold. This enables e.g. to vent the container and/or absorb the small pressure oscillation during the load operation of the plant product; and/or
- the second device comprises or is constituted by a flow deviator or by a valve, preferably a 2-way, e.g. electric or pneumatic, valve; and/or
- the second device is connected directly to the top of the container, where the container remains - in use - empty and there gas accumulates; or the second device is connected to (communicates with) the top of the container by means of a control duct which extends up to the base of the container. The advantage is to place the second (and preferably also the first) device in a place more accessible to a user.

As other variants, the tank for fermenting may comprise
- a pressure sensor for detecting the pressure of a fluid present inside the control duct, in order to determine when an implosion is imminent, and/or
- a liquid detector for detecting the presence of liquid inside the control duct, in order to discard false readings of the pressure sensor due to a liquid column inside the control duct.

In particular, to simplify the management and automate the method with artificial intelligence, the tank for fermenting may comprise a processing unit configured or programmed to
read the pressure sensor to detect a pressure value of the internal pressure of the container, and
drive the second device to selectively put into communication the interior of the container and the first device if the detected pressure value falls or is below a pressure threshold.

To avoid incorrect readings of the pressure sensor, the tank may comprise a liquid sensor to detect the presence of liquid inside the control duct, preferably at the detection point of the pressure sensor, and the processing unit may be configured or programmed to read a data from the liquid sensor and execute a function dependent from such data, for example reporting an anomaly (fluid present) through a sound or light warning. In particular, to avoid an implosion caused by a control duct full of liquid, the tank may comprise a first (optional) valve to isolate the control duct from the top of the container, and a second valve to evacuate the control duct, the processing unit being configured or programmed to close the first valve and open the second valve when the liquid sensor detects liquid.

As a further protection, the tank may comprise
a diaphragm adapted to break if the internal pressure of the container is lower than the atmospheric one,
a normally-open valve, preferably located on the top of the container, which is interposed between the inside of the container and the diaphragm,
wherein the processing unit is configured or programmed to power supply the valve in order to keep it closed,

So, if by a fault the surveillance of the processing unit should fail, the anti-implosion protection of the diaphragm would trigger automatically.

Preferably the diaphragm is comprised in a rupture disk, which may optionally comprise a second diaphragm capable of rupture if the internal pressure of the container increases beyond a threshold. The second diaphragm has the advantage of preventing an overpressure in the container.

Preferably the container is an autoclave.

Another aspect of the invention is a program comprising instructions designed to execute the method when the program is executed by a microprocessor.

Preferred embodiments of the invention will now be described with reference to the attached drawing, wherein
fig. 1 shows a front view of a winemaking tank according to the invention;
fig. 2 shows a front view of a second winemaking fermentation tank according to the invention.

In the drawing same reference numerals indicate same parts, and relative terms such as *vertical, up, top, bottom,* etc. are meant to refer to the tank as in use.

A winemaking tank MC comprises a closed, e.g. cylindrical, vertical-axis container 10, having a bottom 14 and a top 12. The bottom 14 is elevated from the ground by legs 16 and comprises a drain 18 for the liquid contained therein. Note, however, that the invention can be applied similarly on horizontal-type tanks, like e.g. those of cylindrical shape and used with the cylinder axis put horizontally.

Optionally on the container 10 there are mounted:
- an outer level rod 20 to display at the outside the level of liquid inside the container 10. The rod 20 communicates with the bottom 14 and the top 12 of the container;
- a maximum-load duct 24, which extends vertically inside the container 10 and has a hatch on the side of the container 10. It serves to signal the maximum level reached during the filling phase;
- a wash pipe 22 which extends up to the top 12 to further end at a washing head 38 placed inside the container 10;
- a gas control pipe 26, which extends vertically from the top 12 down to about the legs 16. The pipe 26 at the top communicates with the inside of the top 12 and ends at the bottom with a pressure gauge 30, a degassing valve 28 and an unloading tap 29.

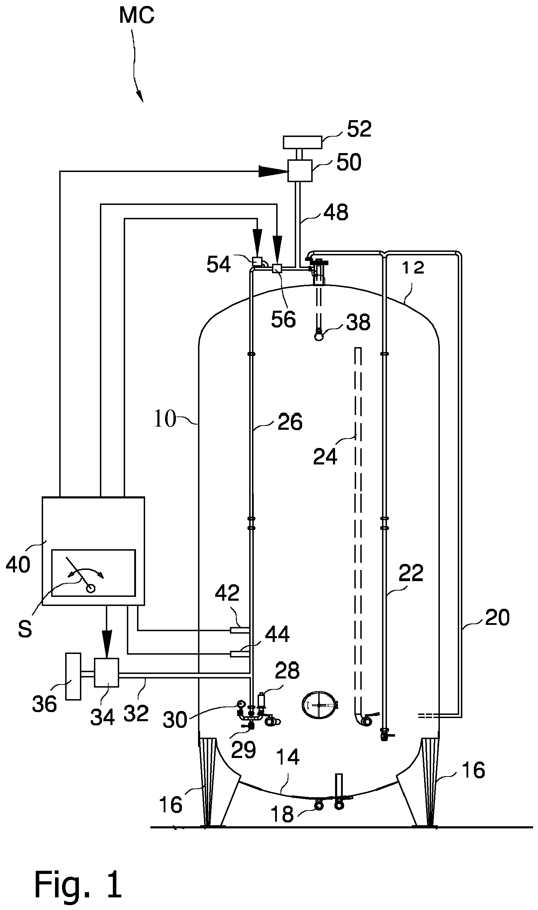

The pipe 26 and/or its accessories 28, 29, 30 are not essential, but give such ease of use to the user (for not having to climb on top 12), which - in practice - are always present. Therefore the example described in Fig. 1 shows a system adapted to the pipe 26.

To the pipe 26 is connected a conduit 32 which comes to a two-way electric or pneumatic valve 34 (or to a similar flow deviator), which is connected to a valve 36. The valve 34 is able to put into communication or isolate the conduit 32 with/from the mechanical valve 36, and the latter is able to open if the external atmospheric pressure is greater than the inner one of the container 10. Optionally, the valve 36 is a double-effect one, i.e. also constructed to open if the pressure inside the container is greater than a threshold (e.g. 100 or 50 millibars).

If the pipe 26 is absent, the fitting 32 may be mounted on the top 12 or on the pipe 22. The valve 34 is preferably driven by an electronic processing unit 40, e.g. a PLC or a microprocessor system. Preferably the unit 40 comprises a selector S, in the form of movable mechanical member or logic variable internal to a program (shown e.g. as/by a button or flag on a display of unit 40). The state or value of the selector S is bi-stable and modifiable e.g. manually by a user, e.g. either through a user interface like a touch-screen or by manipulation. By acting on the selector S the unit 40 is controlled to switch the status of valve 34. Therefore, the tank MC can operate with two operating modes:
1. with the diverter 34 in a first position, the fitting conduit 32 communicates with the valve 36. This prevents the implosion of the container 10 if its internal pressure were to drop below the atmospheric one, in which case the valve 36 would allow the suction of air inside the container 10; and
2. with the diverter 34 in a second position, the fitting conduit 32 is isolated from the valve 36. Thus a high pressure generated or present in the container 10 does not adversely affect or does not damage the valve 36. In fact, a valve such as 36 cannot be so sensitive to switch at pressures of a few negative millibars and then to bear as well a reverse pressure higher by several orders of magnitude.

The following actions related to the control or regulation of the tank MC and MC2 are meant to be performed by the unit 40, e.g. through appropriate program or electronic circuitry.

On the pipe 26 is preferably installed a pressure sensor 42, read and managed by the unit 40. By reading the sensor 42, the unit 40 can automate the control of the diverter S, namely of the valve 34. In particular, with the unit 40 a pressure threshold can be programmed with which to compare the value read from the sensor 42. If the unit 40 calculates that this value is below the threshold (e.g. 50 or 100 millibars), it sets the valve 34 in the first mode, and vice versa. Thus, in fermentative phase, with high pressure in the container 10, the valve 36 is insulated and will not be damaged; while with low or no pressure, during storage or emptying, the valve 36 prevents implosion.

If the level rod 20 breaks or the container 10 is emptied without switching the selector S during the second mode, an accidental implosion can take place. The unit 40 instead intervenes and averts the danger. On the duct or pipe 26 is preferably also installed a liquid sensor 44, read and operated by the unit 40. The sensor 44 has a detection point inside the pipe 26 close to or in correspondence of the detection point of the sensor 42. Knowing whether in the pipe 26 there is liquid or not allows determining whether the value read by the sensor 42 is reliable, or if the sensor 42 is measuring a pressure correctly due to fermentation of gas or due to a column of liquid mistakenly present in the pipe 26. Having the latter to remain empty under normal conditions, the unit 40 may detect the anomaly and warn the user, e.g. by emitting a light or sound signal.

It should be noted that the accidental emptying of the pipe 26 through the tap 29 may implode the container 10 due to siphoning. This does not happen if the pipe 32 by-passes the pipe 26 near the top 12 or by-passes the top 12 itself. Preferably the unit 40 is then connected, to drive them, to:
a valve 56 mounted to isolate the pipe 26 from the container 10, and
to a valve 54 mounted on the pipe 26 to put it into communication with the external environment.

The valve 56 is however optional with respect to the valve 54, being essential only to empty the pipe 26. The unit 40, if it detects liquid by the sensor 44, can automatically close the valve 56 and open the valve 54, in order to allow the emptying of the pipe 26 by manually opening the valve 29, thereby avoiding the implosion. Note that the presence of the valve 34 allows the maintenance of the valve 36 even when the tank 10 is pressurized.

If an accident interrupts the power supply to the unit 40 the anti-implosion control could fail. One solution may be to equip the unit 40 with a battery. Another solution, even more secure and definitive, may be to equip the tank MC with an additional system composed of a valve 50 connected to the pipe 26 by a tube 48 and a break disc 52 connected to the valve 50. The break disc 52 has a diaphragm intended to break if the pressure inside the container 10 is less than the atmospheric pressure and, unlike a vacuum-breaker valve, can be constructed to bear also high overpressure internal to the container 10.

The valve 50 is a normally-open valve which is kept closed by the constant intervention of the unit 40 when it is working, e.g. by an electrical signal or by managing the supply to the valve 50 of a pressurized fluid (oil or compressed air). When the unit 40 is off, the valve 50 is no longer energized and opens, thereby putting the break disk 52 in communication with the container 10. Thus an internal depression of the container 10 at most entails perforation of the diaphragm 10 in the disc 52 but does not damage the tank MC.

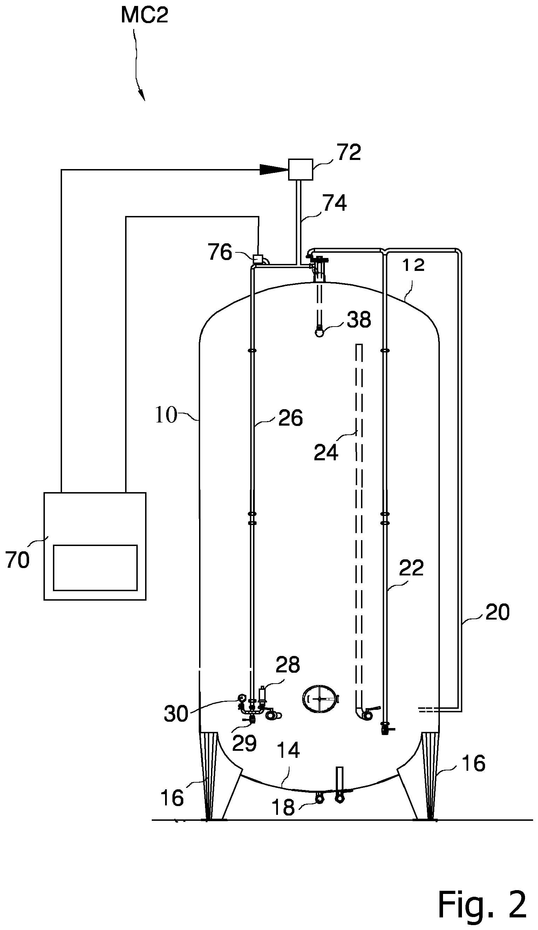

Another variant envisages that the valve 50 is absent on the tube 48. Fig. 2 shows a variant of tank MC2, which comprises the basic structure of the tank MC. The difference is the presence of an electronic unit 70 (like the unit 40) connected to
a pressure sensor 76 placed on the pipe 26 (or on the top 12), and
a valve 72 which is branched off the pipe 26 via a(n) (optional) pipe 74 or branched off the top 12 if the pipe 26 is absent.

Under normal conditions, the valve 72 is closed. The unit 70 reads the sensor 76 for detecting the pressure inside the container 10, and if the sensed pressure is less than a threshold pressure (zero or negative), the unit 70 commands the opening of the valve 72. So implosion is prevented.

Each system of the tank MC is also applicable on the tank MC2, and vice versa.

## Claims

1. Method for reducing the risk of implosion of a tank (10) capable of fermenting a vegetable product in pressurized environment, with the steps of
equipping the tank with a device (36) that allows the entry of outer gas inside the tank if the internal pressure of the tank is lower than that of the external gas with a maximum pressure difference of 50 millibars, and
providing two operation modes of the tank:
- a first mode wherein the device (36) and the tank (10) are in communication with each other to allow the potential entry of gas inside the tank through the device; and
- a second mode wherein the device (36) and the tank (10) are mutually isolated.

2. Method according to claim 1, with the step of acquiring from a user a command of selection of the two operating modes.

3. Method according to claim 1 or 2, with the step of detecting the internal pressure of the tank during the second mode and automatically switch to the first mode if the detected pressure is below a threshold.

4. Method according to claim 1 or 2 or 3, with the step of detecting the internal pressure of the tank during the second mode and generating an alarm signal if the detected pressure is below a threshold.

5. Method according to any one of the preceding claims, with the step of detecting the pressure (i) inside a duct (26) that from the top (12) of the tank extends down to its base or detecting the pressure (ii) on the top of the tank.

6. Method according to claim 5, with the step of detecting/controlling the presence of liquid at the point where the pressure is detected, and in affirmative case evacuating the liquid from the duct to eliminate the contact with the liquid for said point.

7. Method according to any one of the preceding claims, with the step of supplying a normally-open valve (50) to hold it closed, the valve being placed on the top (12) of the tank and interposed between the tank interior and a diaphragm (52) adapted to break if the internal pressure of the tank is lower than that of the external gas.

8. Tank (MC) for fermenting a vegetable product in pressurized environment, comprising
a pressurizable container (10),
a first device (36) for allowing entry of outside air inside the container if the internal pressure of the container is lower than the atmospheric pressure with a maximum pressure difference of 50 millibars,
a second device (34) operable to selectively put in communication or isolate the interior of the container and the first device.

9. Tank according to claim 8, comprising a pressure sensor (42) for detecting the pressure of a fluid present inside a control duct (26) which from the top (12) of the container (10) extends down to the base of the container.

10. Tank according to claim 8 or 9, comprising a processing unit (40) configured or programmed to
read the pressure sensor (42) to detect a pressure value of the internal pressure of the container, and
drive the second device (34) to selectively put into communication the interior of the container and the first device (36) if the detected pressure value falls or is below a pressure threshold.
